# EUROPEAN PATENT SPECIFICATION

(11) **EP 0 682 570 B1**
(45) Date of publication and mention of the grant of the patent: **11.02.1998**
(21) Application number: 93923612.1
(22) Date of filing: 25.10.1993
(51) Int. Cl.: B05B 17/06, A61M 15/00

(54) **DISPENSING APPARATUS**
ABGABEVORRICHTUNG
APPAREIL DE DISTRIBUTION

(30) Priority: 04.11.1992 GB 9223120
(43) Date of publication of application: 22.11.1995
(73) Proprietor: Bespak plc, King's Lynn Norfolk PE30 2JJ (GB)
(72) Inventor: DAVISON, John Robin, Fring, King's Lynn, Norfolk PE31 6SM (GB); BARNES, Paul, King's Lynn, Norfolk, PE32 1RL (GB); BRACE, Geoffrey, Tenax Corporation, Gary, NC 27512 (US)
(74) Representative: Alexander, Thomas Bruce
(86) International application number: PCT/GB93/02194
(87) International publication number: WO 94/09912

(56) References cited:
- EP-A- 0 156 409
- US-A- 3 790 079

## Description

This invention relates to dispensing apparatus for dispensing liquid as an atomised mist and in particular but not exclusively for dispensing medicaments for inhalation therapy.

It is known from GB-A-2240494 to provide dispensing apparatus in which a perforate membrane is vibrated such that liquid in contact with a rear face of the perforate membrane is dispensed from a front face of the perforate membrane as an atomised mist or spray. It is proposed that in the disclosed apparatus a predetermined dose is delivered by operation of a control circuit which regulates the dispensing period during which the perforate membrane is vibrated, the supply of liquid being contained in a chamber of which the perforate membrane constitutes a front wall.

A disadvantage of such an arrangement is that when not in use the liquid within the chamber is open to air through holes in the perforate membrane and may thereby be subject to evaporative losses or become bacteriologically contaminated or otherwise degraded.

A further disadvantage of such an arrangement is that variation in the rate at which liquid is dispensed through the perforate membrane as a mist will result in variation in the total quantity delivered during the dispensing period.

In EP-A-0 156 409 there is described a device for moistening parts of the human body. The device is formed from an ultrasound sprayer which receives water or a similar liquid from a tank. A valve controls the flow of the liquid from the tank to the sprayer and is controlled by a programmed timed switch which stops the ultrasound sprayer operation after the valve has been closed by the same time switch. The inner cross-section of the tube is so selected so that when the valve is closed, due to the capillary action, no water or similar liquid flows to the ultrasound sprayer thereby preventing the forming at the end of the spraying process large water droplets.

In US-A-3 790 079 there is described apparatus and a method for generating a monodispersed aerosol. Liquid is supplied under pressure to an apparatus for generating liquid droplets which is subjected to a vibratory force. The droplets are discharged into a chamber between the apparatus and the outlet and air is also supplied to the chamber under pressure whereby the droplets emanating from the apparatus are dispersed into the air and discharged with the air through the outlet opening.

According to the present invention there is disclosed dispensing apparatus comprising a housing defining a housing outlet, a perforate membrane having a front face exposed at the dispensing outlet and a rear face, vibrating means connected to the housing and means for operating the vibrating means to vibrate the perforate membrane to dispense droplets of the liquid through the perforate membrane as an atomised spray at the outlet, liquid supply means connected to the housing and characterised by the provision of delivery means operable at successive actuations to deliver a respective metered quantity of the liquid from the liquid supply means into contact with the rear face of the perforate membrane and means for operating the vibrating means for an operating period greater than a dispensing period required for the metered quantity to be dispensed through the perforate membrane whereby in use all the dispensed liquid is consumed prior to the next successive actuation of the delivery means, such that the liquid is isolated from the ambient air between successive actuations of the delivery means.

An advantage of such dispensing apparatus is that it facilitates the dispensing of all of the liquid coming into contact with the rear face of the perforate membrane as a single dose. It is thereby possible to avoid contact between liquid and ambient air during periods of non-use between successive actuations. For pharmaceutical preparations this is particularly important since it may obviate the need for the use of preservatives in the liquid and avoids evaporative losses.

Alternatively the liquid supply means may comprise a reservoir containing liquid and the delivery means may comprise a metering pump connected to the reservoir, the metering pump comprising a cylinder defining a chamber and a piston reciprocatably mounted in the cylinder to displace a metered volume of liquid from the chamber at each actuating stroke.

In an alternative apparatus the liquid supply means comprises a pressurised dispensing container within which a supply of liquid is maintained under pressure and the delivery means comprises a metering valve constituting the liquid isolating means and operable to release from the container the metered quantity of liquid.

Preferably the housing comprises an annular member connected peripherally to the vibrating means and defining a central aperture overlaid by the perforate membrane.

Preferably the annular member comprises an upper surface which is concave so as to be downwardly sloping in a radially inward direction when the apparatus is held in use in a preferred operating orientation.

Advantageously the upper surface of the annular member is formed of a liquid repellant material. A liquid droplet deposited on the upper surface is thereby encouraged to move radially inwardly into contact with the perforate membrane.

Conveniently the annular member comprises a radially innermost surface portion defining the aperture and co-operating with the perforate membrane to define a well for receiving in use a droplet of liquid to be dispensed.

The housing may define a duct communicating between an air inlet and an outlet port, the dispensing outlet being located in the duct intermediate the air inlet and the outlet port such that the front face of the perforate member is exposed to air within the duct.

The outlet port may be an inhalation port for oral or nasal use.

Such an arrangement is useful in the administration of inhaled pharmaceutical liquid products where it is required for an atomised spray of liquid to be entrained in an inhaled air flow passing through the duct.

Apparatus in accordance with the present invention may be provided with an air impeller connected to the housing so as to be operable to create a flow of air through the duct from the air inlet to the outlet port.

Such an arrangement may be used for dispensing cosmetics and perfumes. A further application of such an arrangement is ophthalmic use where an ophthalmic preparation can be used to generate an atraumatic mist which is administered to the user's eye by means of an eye cup provided in the housing so as to communicate with the duct.

The means for operating the vibrating means preferably comprises a timer connected to the vibrating means and operable to determine the operating period during which the vibrating means is actuated, when the operating period is selected to be greater than the dispensing period required for the metered quantity to be dispensed through the membrane.

In a further preferred embodiment the liquid supply means comprises a plurality of cells each containing a metered volume of liquid and the delivery means comprises a dispensing station operable to release at each actuation thereof the contents of a respective cell.

Particular embodiments of the present invention will now be described by way of example only and with reference to the accompanying drawings in which:-
Figure 1 is a schematic sectioned elevation of a first dispensing apparatus in accordance with the present invention;
Figure 2 is a schematic sectioned elevation of an alternative dispensing apparatus having a pipette with an indexing mechanism;
Figure 3 is a schematic sectioned elevation of a further alternative dispensing apparatus having a metering pump;
Figure 4 is a schematic sectioned elevation of a further alternative dispensing apparatus having an electrically driven pump;
Figure 5 is a schematic sectioned elevation of a further alternative dispensing apparatus having a pressurised dispensing container with a metering valve;
Figure 6 is a schematic sectioned elevation of a further alternative dispensing apparatus in which the liquid supply means comprises a plurality of cells whose respective contents are released during successive actuations at a dispensing station;
Figure 7 is a schematic sectioned elevation of a further alternative dispensing apparatus for use in dispensing an ophthalmic preparation; and
Figure 8 is a schematic sectioned elevation of a further alternative dispensing apparatus having an air impeller for dispensing cosmetic preparations.

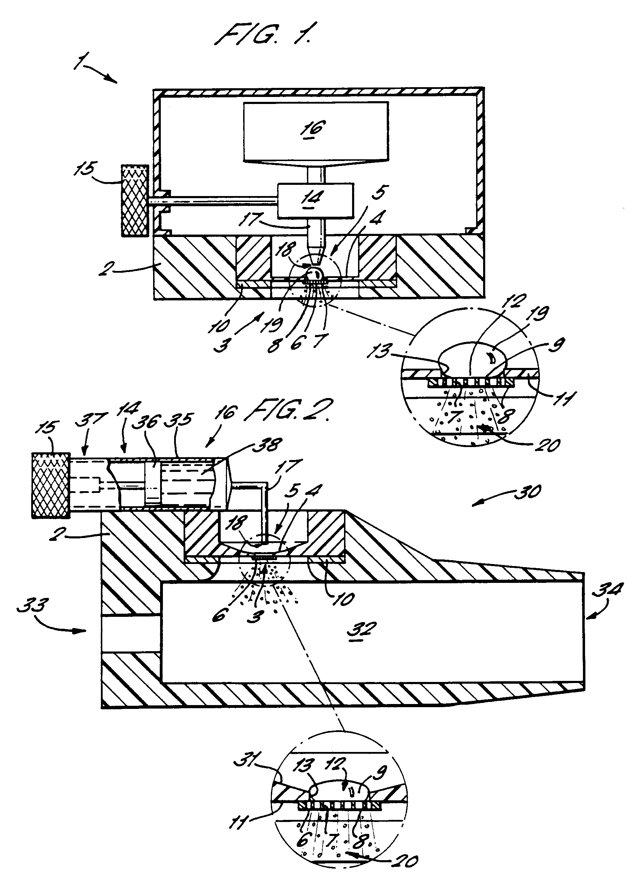

In Figure 1 a first dispensing apparatus 1 has a housing 2 defining a dispensing outlet 3. The housing 2 includes an annular member 4 defining a central aperture 5 which is overlaid by a perforate membrane 6.

The perforate membrane 6 is peripherally bonded to the annular member 4 so as to close the aperture 5 and defines an array of holes 7 of 3 microns diameter and 25 microns spacing.

The annular member 4 extends horizontally such that a front face 8 of the perforate membrane 6 is downwardly oriented and a rear face 9 of the perforate membrane is upwardly directed.

The annular member 4 extends radially with uniform thickness into contact with an annular piezoelectric transducer 10 which is connected to an oscillator circuit (not shown) operable to energise the transducer. The transducer 10 is arranged such that vibration is applied at right angles to the plane of the annular member 4 such that transverse acoustic waves are propagated radially inwardly of the annular member so as to excite the perforate membrane 6 in an axial direction i.e. producing vertical movement of the membrane.

The perforate membrane 6 is bonded to a lower surface 11 of the annular member 4 such that, because of the finite thickness of the member at the aperture 5, a shallow well 12 is defined in the aperture 5 above the perforate membrane 6 and bounded radially by an inner vertical annular face 13 of the annular member 4.

A delivery means 14 is located within the housing 2 and is operable by means of an actuator 15 to deliver at each actuation a metered volume of liquid from a liquid supply means 16, the liquid being delivered through a delivery tube 17 to a delivery location 18 immediately adjacent and above the perforate membrane 6.

A droplet of liquid 19 is shown located on top of the perforate member 6 within the well 12 immediately following actuation of the delivery means 14 to dispense a metered volume of 20 microlitres of liquid, the perforate membrane being vibrated to produce an atomised spray 20 in the air surrounding the dispensing outlet 3.

In use, the perforate membrane 6 is initially free from liquid and a metered volume is dispensed by actuation of the delivery means 15 such that a droplet of liquid is placed in the well 12. The vibrating means is then actuated and continues to vibrate the perforate membrane 6 for an operating period determined by a timer (not shown).

The operating period is selected to be greater than the dispensing period required to dispense all of the liquid contained in the droplet 19 as an atomised spray or mist through the perforate membrane holes 7.

Since in practice the dispensing period required to totally dispense a metered volume will vary from actuation to actuation, the operating period is selected to be sufficiently greater than the average dispensing period in order to accommodate such variation thereby ensuring that the quantity of liquid dispensed is independent of the rate at which the liquid flows through the perforate membrane.

The vibrating means is the de-actuated until further use is required.

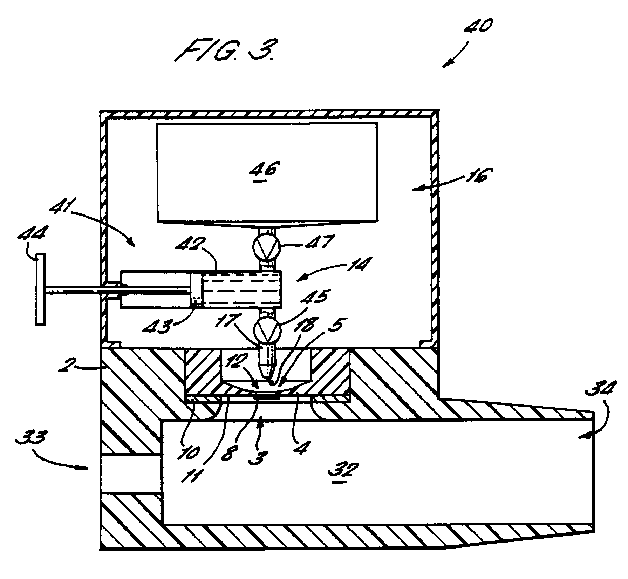

An alternative dispensing apparatus 30 shown in Figure 2 will now be described using corresponding reference numerals to those of Figure 1 where appropriate for corresponding element.

The second dispensing apparatus 30 includes a housing 2 with an annular member 4 supporting a perforate membrane 6 for vibration by actuation of a transducer 10. The annular member 4 has an upper surface 31 which is concave and downwardly sloping in a radially inward direction so that the aperture 5 is located at the lowest part of the surface 31 thereby assisting draining of any liquid on the upper surface 31 into the well 12.

The upper surface 31 is coated with a liquid repellant surface to assist this draining action and ensure that any droplet of liquid deposited on the upper surface finds its way into contact with the perforate membrane 6.

The housing 2 defines a horizontally extending cylindrical duct 32 communicating between an air inlet 33 and an inhalation port 34 suitable for oral inhalation. The outlet 3 is located intermediate the inlet 33 and the inhalation port 34 so as to communicate with the duct 32, the perforate membrane 6 having a front face 8 which is directly exposed to air within the duct 32.

The second dispensing apparatus 30 has a delivery means 14 comprising a pipette 35 within which a piston 36 is advanceable by means of an indexing mechanism 37 in discrete measured steps so as to displace metered volumes of liquid 38 from the pipette. A delivery tube 17 communicates between the pipette 35 and a delivery location 18 so as to dispense a metered quantity of the liquid into contact with the perforate membrane 6 at each actuation of the indexing mechanism 37.

In use the indexing mechanism is actuated to deliver a drop into the well 12 and the vibrating means is then actuated. Vibration of the perforate membrane 6 discharges a fine mist 20 of droplets into the duct 32 until such time as the liquid within the well is entirely consumed. The mist is entrained in an air flow within the duct when the user inhales air through the inhalation port 34 such that the liquid is orally administered for inhalation into the user's lungs.

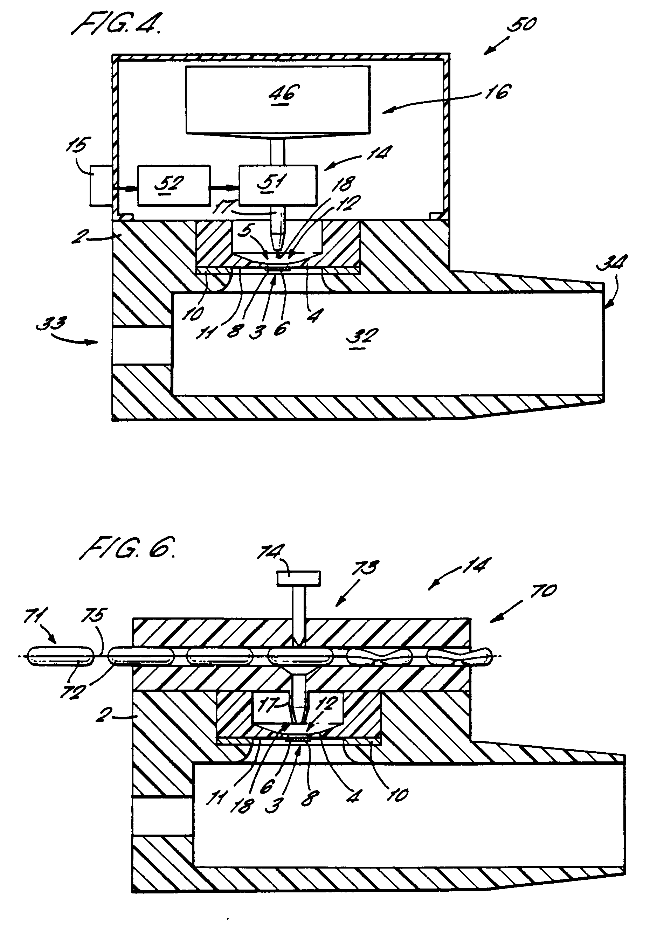

A third dispensing apparatus 40 is shown in Figure 3 and will now be described using corresponding reference numerals to those of preceding Figures where appropriate for corresponding elements.

A third dispensing apparatus 40 includes a housing 2, annular member 4 and duct 32 of the type described above with reference to the second apparatus 30 in Figure 2. The third dispensing apparatus 40 differs in that the delivery means 14 is a manually actuated metering pump 41 which has a cylinder 42 receiving a piston 43 actuated by means of an actuator 44. At each actuation a single stroke of the piston 43 displaces from the cylinder 42 a metered quantity of liquid which flows through a one-way outlet valve 45 into delivery tube 17. The piston then returns to its rest position and the chamber is refilled for further operation. The cylinder 42 and piston 43 are shown schematically in Figure 3 and not to scale, the appropriate stroke and metered volume in a practical arrangement being selected to dispense a drop of liquid at each actuation.

Liquid is supplied to the pump 41 from a reservoir 46 via an inlet valve 47, the reservoir being of sufficient capacity to contain a large number of metered volumes of liquid.

In use the metering pump 41 is actuated by manual depression of the actuator 44 and a single stroke of the piston 43 displaces a droplet of liquid on to the perforate membrane 6 via the delivery tube 17.

The transducer 10 is then actuated so as to vibrate the perforate membrane and dispense atomised droplets into the duct 32 from where they are inhaled by the user via the inhalation port 34. After a time period sufficient for the droplet to be consumed the transducer 10 is de-actuated and the dispensing apparatus is ready for further use.

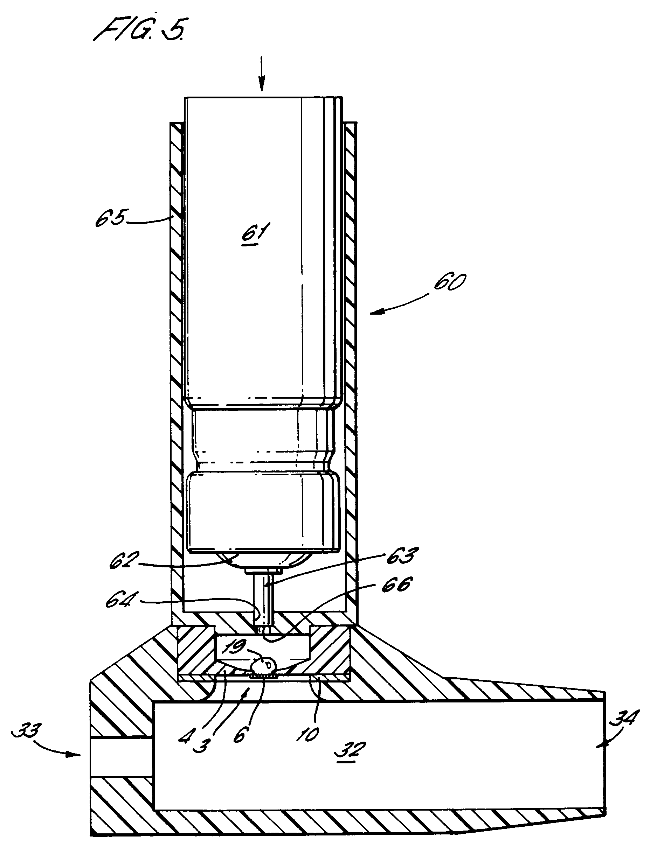

A fourth alternative dispensing apparatus 50 shown in Figure 4 will now be described using corresponding reference numerals to those of preceding Figures where appropriate for corresponding elements.

The dispensing apparatus 50 is generally similar to the third dispensing apparatus 40 of Figure 3 but includes an electrically operated pump 51 which is operable to deliver liquid from a reservoir 46 to a delivery location 18 when energised by a controller circuit 52.

The pump 51 is a peristaltic pump providing a continuous output flow when energised and the controller circuit 52 achieves delivery of a measured volume by controlling the duration for which pump 51 is energised.

Once a metered volume of liquid has been delivered on the perforate membrane 6 the operation of the dispensing apparatus 50 corresponds to that of the third dispensing apparatus 40 described above.

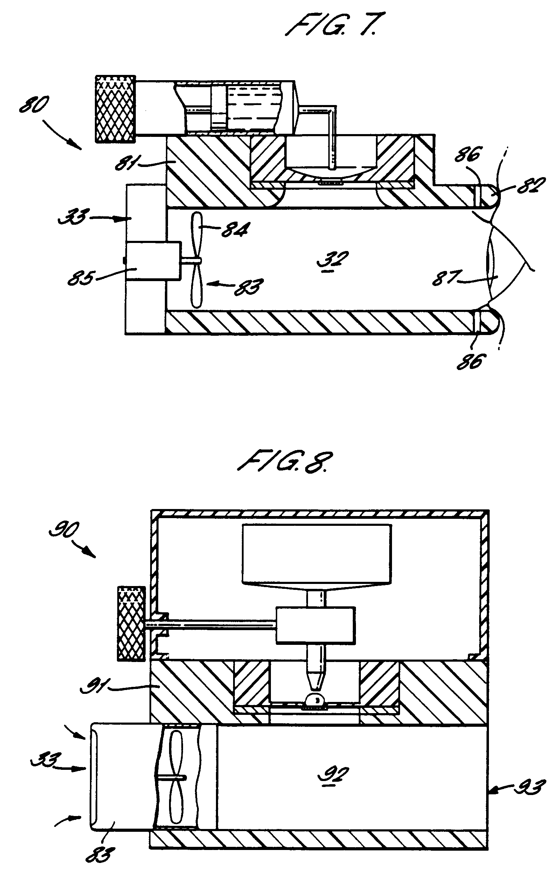

A fifth dispensing apparatus 60 shown in Figure 5 will now be described using corresponding reference numerals to those of preceding Figures where appropriate for corresponding elements.

The fifth dispensing apparatus 60 includes a housing 2 with an annular member 4 supporting perforate membrane 6 of the same general type as those described above with reference to Figures 2, 3 and 4. A quantity of liquid is contained within a pressurised dispensing container 61 of a type commonly utilised in aerosol dispensers. The container 61 has a metering valve 62 operable to dispense a metered volume of liquid through a tubular stem 63 in response to depression of the stem 63 relative to the container 61.

The stem 63 is received within a socket 64 fixed to the housing 2 and the container 61 is displaceable relative to a tubular sheath 65 extending upwardly of the housing so as to provide relative movement between the container and the stem 63 thereby enabling the metering valve 62 to be actuated by manual depression of the container 61 relative to the housing 2.

The socket 64 defines a delivery orifice 66 communicating with the stem 63 and through which the liquid is dispensed on to the perforate membrane 6.

In use the perforate membrane is vibrated by actuation of the transducer 10 for a period sufficient to totally atomise a droplet of liquid received within the well 12 so that a fine mist can be orally inhaled via the duct 32.

A sixth dispensing apparatus 70 is shown in Figure 6 and will now be described using corresponding references to those of preceding Figures where appropriate for corresponding elements.

The sixth dispensing apparatus 70 includes a housing 2, an annular member 4 and a perforate membrane 6 of the type disclosed with reference to Figures 2 and 5. A delivery means 14 comprises a liquid supply means 71 in which metered quantities of liquid are contained within individual cells 72. The cells 72 are fed in turn to a dispensing station 73 which is operable by depression of an actuator 74 to release the contents of a cell and conduct the contents of the cell via delivery tube 17 on the perforate membrane 6.

The cells 72 comprise gelatin capsules formed on a disposable strip 75 which is fed to the dispensing station 73 where the capsules are ruptured by action of the actuator 74 during each dispensing operation.

A seventh dispensing apparatus 80 is shown in Figure 7 and will now be described using corresponding references to those of preceding Figures where appropriate for corresponding elements.

The seventh dispensing apparatus 80 is based on the second apparatus 30 of Figure 2 but includes a modified housing 81 in which the inhalation port 34 of Figure 2 is replaced by an eye cup 82 and air inlet 33 is provided with an air impeller 83.

The air impeller 83 consists of a fan 84 driven by an electric motor 85 so as to create a flow of air along duct 32 and air release holes 86 are provided adjacent to the eye cup 82 to allow the escape of air peripherally of the eye cup.

The seventh dispensing apparatus 80 is operable to dispense an aerosol mist into the duct 32 in the same manner as the second dispensing apparatus 30 of Figure 2. The product dispensed as a liquid is an ophthalmic preparation providing an atraumatic mist delivered to the user's eye 87 when engaged with the eye cup 82.

An eighth dispensing apparatus 90 is shown in Figure 8 and will now be described using corresponding references to those of preceding Figures where appropriate for corresponding elements.

The eighth dispensing apparatus 90 is based on the first apparatus 1 of Figure 1 but includes a modified housing 91 which defines a cylindrical duct into which the dispensing outlet 3 opens to release an atomised spray in use. The duct 92 has an outlet 93 through which air emerges when a flow of air through the duct 92 is created by an air impeller 83 located in the air inlet 33. The air impeller 83 comprises a fan 84 driven by an electric motor.

The eighth dispensing apparatus 90 is primarily intended for dispensing cosmetic preparations, perfumes and fragrances.

The dispensing apparatus shown in Figure 1 may be used in applications other than for medical inhalation therapy such as to dispense cosmetic preparations including perfume.

The dispensing apparatus of Figures 2 to 6 is suitable for inhalation therapy and may in each case be modified to include an inhalation sensor in order to synchronise the release of droplets through the perforate membrane with inhalation of the user. The inhalation port may be adapted for oral or nasal inhalation.

The fourth dispensing apparatus shown in Figure 4 with reference to a peristaltic pump may alternatively use a piezoelectric micro pump of the type disclosed in EP-0398583A in which a perforate membrane is reciprocated by action of a piezoelectric transducer.

The seventh dispensing apparatus 80 of Figure 7 may alternatively be provided with a metering pump of the type disclosed with reference to Figure 3 or Figure 4 or alternatively may utilise the pressurised dispensing container 61 and metering valve 62 of the fifth dispensing apparatus shown in Figure 5. Alternatively the liquid supply means 71 of Figure 6 may be utilised in the seventh dispensing apparatus 80.

Similar comments apply to the eighth dispensing apparatus 90 where similarly alternative arrangements may be utilised to deliver a droplet on to the perforate membrane 6.

The air impeller 83 utilised in Figures 7 and 8 may be replaced by an equivalent arrangement such as a mechanically driven fan or bellows arrangement.

The fifth dispensing apparatus 70 may alternatively be used with different types of package defining individual cells. For example a plastic, aluminium or laminated strip may be formed so as to define pockets each defining a cell containing a pre-measured quantity of liquid, each pocket being closed by a second strip of plastic, aluminium foil or a laminate. The liquid may then be released by piercing the pocket or by peeling off the second strip.

In the configuration shown in Figure 6, individual metered quantities of liquid are placed in the packaging at the time of manufacture. Each dose is consumed at each actuation of the dispensing apparatus. This arrangement has the advantage of providing for different doses to be administered by the dispensing apparatus by providing a range of packaged doses of predetermined volume from which the required dose can be selected or prescribed as may be required.

The frequency vibration of the perforate membrane described above with reference to each of the embodiments may be selected to any suitable value, preferably in the kilohertz or megahertz range.

The pressurised dispensing container 61 of Figure 5 may be of the conventional type in which an evaporable propellant is mixed with a liquid product or of an alternative type such as a dispensing container 61 adapted to dispense under pressure from a compressed gas. The liquid product may be contained separately from the propellant liquid or gas within a collapsible bag or compartment.

## Claims

1. Dispensing apparatus (1) comprising a housing (2) defining a dispensing outlet (3), a perforate membrane (6) having a front face (8) exposed at the dispensing outlet and a rear face (9), vibrating means (10) connected to the housing and means for operating the vibrating means to vibrate the perforate membrane to dispense droplets of liquid through the perforate membrane as an atomised spray at the outlet, liquid supply means (16) connected to the housing and characterised by the provision of delivery means (14) operable at successive actuations to deliver a respective metered quantity (19) of the liquid from the liquid supply means into contact with a rear face of the perforate membrane and means for operating the vibrating means for an operating period greater than a dispensing period required for the metered quantity to be dispensed through the perforate membrane whereby in use all of the dispensed liquid is consumed prior to the next successive actuation of the delivery means, such that the liquid is isolated from the ambient air between successive actuations of the delivery means.

2. Dispensing apparatus as claimed in claim 1 wherein the liquid supply means comprises a reservoir (16) containing liquid and the delivery means comprises a metering pump (14) connected to the reservoir, the metering pump comprising a cylinder (42) defining a chamber and a piston (43) reciprocatably mounted in the cylinder to displace a metered volume of liquid from the chamber at each actuating stroke.

3. Dispensing apparatus as claimed in claim 1 wherein the liquid supply means comprises a pressurised dispensing container (61) within which a supply of liquid is maintained under pressure and the delivery means comprises a metering valve (62) constituting the liquid isolating means and operable to release from the container the metered quantity of liquid.

4. Dispensing apparatus as claimed in any preceding claim wherein the housing comprises an annular member (4) connected peripherally to the vibrating means and defining a central aperture (5) overlaid by the perforate membrane.

5. Dispensing apparatus as claimed in claim 4 wherein the annular member comprises an upper surface which is concave so as to be downwardly sloping in a radially inward direction when the apparatus is held in use in a preferred operating orientation.

6. Dispensing apparatus as claimed in claim 5 wherein the upper surface of the annular member is formed of a liquid repellant material.

7. Dispensing apparatus as claimed in any of claims 5 and 6 wherein the annular member comprises a radially innermost surface portion (13) defining the aperture and co-operating with the perforate membrane to define a well (12) for receiving in use a droplet (19) of liquid constituting the metered quantity of liquid to be dispensed.

8. Dispensing apparatus as claimed in any preceding claim wherein the housing defines a duct (32) communicating between an air inlet (33) and an outlet port (34), the dispensing outlet (3) being located in the duct intermediate the air inlet and the outlet port such that the front face of the perforate membrane is exposed to air within the duct.

9. Dispensing apparatus as claimed in claim 8 wherein an air impeller (83) is connected to the housing so as to be operable to create a flow of air through the duct from the air inlet to the outlet port.

10. Dispensing apparatus as claimed in claim 9 wherein the housing defines an eye cup (82) communicating with the duct.

11. Dispensing apparatus as claimed in claim 8 wherein the outlet port comprises an inhalation port (34) for oral or nasal use.

12. Dispensing apparatus as claimed in any preceding claim wherein the means for operating the vibrating means comprises a timer connected to the vibrating means and operable to determine the operating period during which the vibrating means is actuated and wherein the operating period is selected to be greater than the dispensing period required for the metered quantity to be dispensed through the perforate membrane.

13. A method of dispensing liquid as an atomised spray comprising the steps of delivering a metered quantity of liquid from a liquid supply means (16) into contact with a rear face (9) of a perforate membrane (6), vibrating the perforate membrane (6) such that liquid is dispensed through the perforate membrane (6) as an atomised spray, characterised in that the perforate membrane (6) is initially free from liquid and the liquid is isolated from the ambient air between successive deliveries of liquid and in that the perforate membrane (6) is vibrated for an operating period greater than a dispensing period required for the metered quantity to be fully dispensed.

14. Dispensing apparatus as claimed in claim 1 wherein the liquid supply means comprises a plurality of cells (72) each containing a metered volume of liquid and the delivery means comprises a dispensing station (73) operable to release at each actuation thereof the contents of a respective cell, and means for operating the vibrating means for an operating period greater than a dispensing period required for the metered quantity to be dispensed through the perforate membrane whereby in use all of the dispensed liquid is consumed prior to the next successive actuation of the delivery means, such that the liquid is isolated from the ambient air between successive actuations of the delivery means.

## Patentansprüche

1. Abgabevorrichtung (1) umfassend:
ein Gehäuse (2), welches einen Abgabeauslaß (3) begrenzt,
eine perforierte Membran (6) mit einer zu dem Abgabeauslaß freiliegenden Vorderseite (8) und einer Rückseite (9),
mit dem Gehäuse verbundene Vibrationsmittel (10) und Mittel zum Betreiben der Vibrationsmittel zum Vibrieren der perforierten Membran, um Flüssigkeitströpfchen durch die perforierte Membran als einen zerstäubten Sprühnebel an dem Auslaß abzugeben,
Flüssigkeitsvorratsmittel (16), welche mit dem Gehäuse verbunden sind,
**dadurch gekennzeichnet,**
daß Zuführmittel (14) vorgesehen sind, die bei aufeinanderfolgenden Betätigungen betreibbar sind, um eine jeweilige dosierte Menge (19) der Flüssigkeit aus den Flüssigkeitsvorratsmitteln in Kontakt mit einer Rückseite der perforierten Membran zu bringen,
sowie Mittel vorgesehen sind, zum Betreiben der Vibrationsmittel während einer Betriebszeitdauer, die länger als eine Abgabezeitdauer ist, die zur Abgabe der dosierten Menge durch die perforierte Membran erforderlich ist, wodurch im Gebrauch die gesamte abgegebene Flüssigkeit, vor der nächstfolgenden Betätigung der Zuführmittel, verbraucht wird , so daß die Flüssigkeit von der Umgebungsluft zwischen aufeinanderfolgenden Betätigungen der Zuführmittel isoliert ist.

2. Abgabevorrichtung nach Anspruch 1, wobei die Flüssigkeitsvorratsmittel einen Flüssigkeit enthaltenden Behälter (16) umfassen und die Zuführmittel eine mit dem Behälter verbundene Dosierpumpe (14) umfassen, wobei die Dosierpumpe einen eine Kammer begrenzenden Zylinder (42) und einen Kolben (43) umfaßt, der in dem Zylinder hin- und herbewegbar angebracht ist, um bei jedem Betätigungshub ein dosiertes Volumen an Flüssigkeit aus der Kammer zu verdrängen.

3. Abgabevorrichtung nach Anspruch 1, wobei die Flüssigkeitsvorratsmittel einen unter Druck stehenden Abgabebehälter (61) umfassen, in welchem ein Vorrat an Flüssigkeit unter Druck gehalten ist, und die Zuführmittel ein Dosierventil (62) umfassen, welches die Flüssigkeitsisoliermittel bildet und zur Abgabe der dosierten Menge an Flüssigkeit aus dem Behälter betreibbar ist.

4. Abgabevorrichtung nach einem der vorangegangenen Ansprüche, wobei das Gehäuse ein ringförmiges Element (4) umfaßt, welches am Umfang mit den Vibrationsmitteln verbunden ist und eine zentrale Öffnung (5) begrenzt, welche durch die perforierte Membran bedeckt wird.

5. Abgabevorrichtung nach Anspruch 4, wobei das ringförmige Element eine obere Fläche umfaßt, welche konkav ist, so daß sie in einer Radialeinwärts-Richtung nach unten geneigt ist, wenn die Vorrichtung im Gebrauch in einer bevorzugten Betriebsorientierung gehalten wird.

6. Abgabevorrichtung nach Anspruch 5, wobei die obere Fläche des ringförmigen Elements aus einem flüssigkeitsabstoßenden Material gebildet ist.

7. Abgabevorrichtung nach einem der Ansprüche 5 und 6, wobei das ringförmige Element einen radial innersten Oberflächenabschnitt (13) umfaßt, welcher die Öffnung begrenzt und mit der perforierten Membran zusammenwirkt, um einen Schacht (12) zu begrenzen, um während des Gebrauchs ein Flüssigkeitströpfchen (19) aufzunehmen, welches die dosierte Menge der abzugebenden Flüssigkeit bildet.

8. Abgabevorrichtung nach einem der vorangegangenen Ansprüche, wobei das Gehäuse einen Kanal (32) begrenzt, welcher einen Lufteinlaß (33) und eine Auslaßöffnung (34) miteinander verbindet, wobei der Abgabeauslaß (3) in dem Kanal, zwischen dem Lufteinlaß und der Auslaßöffnung, derart angeordnet ist, daß die Vorderseite der perforierten Membran der Luft in dem Kanal ausgesetzt ist.

9. Abgabevorrichtung nach Anspruch 8, wobei ein Luft-Impeller (83) derart mit dem Gehäuse verbunden ist, daß er zur Erzeugung eines Luftstroms durch den Kanal, von dem Lufteinlaß zu der Auslaßöffnung, betreibbar ist.

10. Abgabevorrichtung nach Anspruch 9, wobei das Gehäuse einen Augenaufsatz (82) begrenzt, welcher mit dem Kanal in Verbindung steht.

11. Abgabevorrichtung nach Anspruch 8, wobei die Auslaßöffnung eine Inhalationsöffnung (34) für oralen oder nasalen Gebrauch umfaßt.

12. Abgabevorrichtung nach einem der vorangegangenen Ansprüche, wobei die Mittel zum Betreiben der Vibrationsmittel einen mit den Vibrationsmitteln verbundenen Zeitgeber umfassen, der betreibbar ist, um die Betriebszeitdauer, während der die Vibrationsmittel betrieben werden, festzulegen, und wobei die Betriebszeitdauer so gewählt ist, daß sie länger als die Abgabezeitdauer ist, die zur Abgabe der dosierten Menge durch die perforierte Membran erforderlich ist.

13. Verfahren zur Abgabe von Flüssigkeit als zerstäubter Sprühnebel, umfassend die Schritte:
Zuführen einer dosierten Menge an Flüssigkeit aus einem Flüssigkeitsvorratsmittel (16) in Kontakt mit einer Rückseite (9) einer perforierten Membran,
Vibrieren der perforierten Membran (6), so daß Flüssigkeit als ein zerstäubter Sprühnebel durch die perforierte Membran (6) abgegeben wird,
**dadurch gekennzeichnet, daß**
die perforierte Membran (6) anfänglich frei von Flüssigkeit ist und die Flüssigkeit zwischen aufeinanderfolgenden Zuführungen von Flüssigkeit von der Umgebungsluft isoliert wird,
und dadurch, daß die perforierte Membran (6) während einer Betriebszeitdauer vibriert wird, die länger ist als die Abgabezeitdauer, die zur vollständigen Abgabe der dosierten Menge benötigt wird.

14. Abgabevorrichtung nach Anspruch 1, wobei die Flüssigkeitsvorratsmittel eine Vielzahl von Zellen (72) umfassen, von denen jede ein dosiertes Volumen an Flüssigkeit enthält, und die Zuführmittel eine Abgabestation (73) umfassen, welche bei jeder Betätigung derselben den Inhalt einer jeweiligen Zelle freigeben kann, und Mittel zum Betreiben der Vibrationsmittel während einer Betriebszeitdauer umfassen, die länger ist als eine Abgabezeitdauer, die benötigt wird, um die dosierte Menge durch die perforierte Membran abzugeben, wodurch im Gebrauch, vor der nächstfolgenden Betätigung der Zuführmittel, die gesamte abgegebene Flüssigkeit verbraucht wird, so daß die Flüssigkeit zwischen aufeinanderfolgenden Betätigungen der Zuführmittel von der Umgebungsluft isoliert ist.

## Revendications

1. Appareil distributeur (1) comprenant un boîtier (2) définissant une sortie de distribution (3), une membrane perforée (6) comportant une face avant (8) exposée à la sortie de distribution et une face arrière (9), un moyen vibrant (10) relié au boîtier et un moyen pour actionner le moyen vibrant afin de faire vibrer la membrane perforée pour distribuer des gouttelettes de liquide via la membrane perforée en un spray atomisé à la sortie, un moyen d'alimentation en liquide (16) relié au boîtier et caractérisé en ce qu'est prévu un moyen de décharge (14) adapté à des actionnements successifs pour décharger une quantité mesurée respective (19) du liquide depuis le moyen d'alimentation en liquide en contact avec une face arrière de la membrane perforée et un moyen pour actionner le moyen vibrant pendant une durée d'actionnement supérieure à une durée de distribution requise pour la quantité mesurée à distribuer via la membrane perforée de manière que, à l'utilisation, la totalité du liquide distribué soit consommée avant l'actionnement suivant du moyen de décharge, afin que le liquide soit isolé de l'air ambiant entre les actionnements successifs du moyen de décharge.

2. Appareil distributeur selon la revendication 1, dans lequel le moyen d'alimentation en liquide comprend un réservoir (16) contenant du liquide et le moyen de décharge comprend une pompe de dosage (14) reliée au réservoir, la pompe de dosage comprenant un cylindre (42) définissant une chambre et un piston (43) monté de manière à aller et venir dans le cylindre pour déplacer un volume mesuré de liquide depuis la chambre à chaque course d'actionnement.

3. Appareil distributeur selon la revendication 1, dans lequel le moyen d'alimentation en liquide comprend un récipient distributeur pressurisé (61) au sein duquel une quantité de liquide est maintenue sous pression et le moyen de décharge comprend une soupape de dosage (62) constituant le moyen d'isolation de liquide et adaptée à libérer du récipient la quantité mesurée de liquide.

4. Appareil distributeur selon l'une quelconque des revendications précédentes, dans lequel le boîtier comprend un organe annulaire (4) relié à sa périphérie au moyen vibrant et définissant une ouverture centrale (5) recouverte par la membrane perforée.

5. Appareil distributeur selon la revendication 4, dans lequel l'organe annulaire comprend une surface supérieure qui est concave afin d'être inclinée vers le bas dans une direction radialement vers l'intérieur lorsque l'appareil est tenu, à l'utilisation, dans une orientation d'actionnement préféree.

6. Appareil distributeur selon la revendication 5, dans lequel la surface supérieure de l'organe annulaire est faite d'un matériau repoussant les liquides.

7. Appareil distributeur selon l'une quelconque des revendications 5 et 6, dans lequel l'organe annulaire comprend une portion de surface la plus interne radialement (13) définissant l'ouverture et coopérant avec la membrane perforée pour définir un puits (12) pour recevoir, à l'utilisation, une gouttelette (19) de liquide constituant la quantité mesurée de liquide à distribuer.

8. Appareil distributeur selon l'une quelconque des revendications précédentes, dans lequel le boîtier définit un conduit (32) communiquant entre une entrée d'air (33) et un orifice de sortie (34), la sortie de distribution (3) étant située dans le conduit entre l'entrée d'air et l'orifice de sortie de manière que la face avant de la membrane perforée soit exposée à l'air au sein du conduit.

9. Appareil distributeur selon la revendication 8, dans lequel une roue à air (83) est reliée au boîtier afin d'être adaptée à créer un flux d'air via le conduit de l'entrée d'air à l'orifice de sortie.

10. Appareil distributeur selon la revendication 9, dans lequel le boîtier définit une coupelle oculaire (82) communicant avec le conduit.

11. Appareil distributeur selon la revendication 8, dans lequel l'orifice de sortie comprend un orifice d'inhalation (34) pour usage oral ou nasal.

12. Appareil distributeur selon l'une quelconque des revendications précédentes, dans lequel le moyen pour actionner le moyen vibrant comprend une minuterie reliée au moyen vibrant et adaptée à déterminer la durée d'actionnement durant laquelle le moyen vibrant est actionné et dans lequel la durée d'actionnement est sélectionnée de manière à être supérieure à la durée de distribution requise pour la quantité mesurée à distribuer via la membrane perforée.

13. Procédé de distribution de liquide en un spray atomisé comprenant les phases consistant à distribuer une quantité mesurée de liquide depuis un moyen d'alimentation en liquide (16) en contact avec une face arrière (9) d'une membrane perforée (6), faire vibrer la membrane perforée (6) de manière que le liquide soit distribué via la membrane perforée (6) en un spray atomisé, caractérisé en ce que la membrane perforée (6) est initialement exempte de liquide et le liquide est isolé de l'air ambiant entre les décharges successives de liquide et en ce que la membrane perforée (6) est soumise à des vibrations pendant une durée d'actionnement supérieure à une durée de distribution requise pour que la quantité mesurée soit totalement distribuée.

14. Appareil distributeur selon la revendication 1, dans lequel le moyen d'alimentation en liquide comprend une pluralité d'alvéoles (72) contenant chacun un volume mesuré de liquide et le moyen de décharge comprend un poste distributeur (73) adapté à libérer à chaque actionnement le contenu d'un alvéole respectif, et un moyen pour actionner le moyen vibrant pendant une durée d'actionnement supérieure à une durée de distribution requise pour que la quantité mesurée soit distribuée via la membrane perforée de manière que, à l'utilisation, la totalité du liquide distribué soit consommée avant l'actionnement suivant du moyen de décharge, afin que le liquide soit isolé de l'air ambiant entre les actionnements successifs du moyen de décharge.
